(19) Europäisches Patentamt
European Patent Office
Office européen des brevets

(11) **EP 2 266 674 A1**

(12) **EUROPÄISCHE PATENTANMELDUNG**

(43) Veröffentlichungstag:
**29.12.2010 Patentblatt 2010/52**

(51) Int Cl.:
**B01D 3/14** *(2006.01)*   **B01D 3/32** *(2006.01)*
**C07C 7/04** *(2006.01)*

(21) Anmeldenummer: **10165920.9**

(22) Anmeldetag: **15.06.2010**

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(30) Priorität: **22.06.2009 EP 09163359**

(27) Früher eingereichte Anmeldung:
**22.06.2009 EP 09163359**

(71) Anmelder: **BASF SE**
**67056 Ludwigshafen (DE)**

(72) Erfinder:
• **Kaibel, Gerd Dr.**
  **68623 Lampertheim (DE)**
• **Müller, Christian**
  **68167 Mannheim (DE)**
• **Hugo, Randolf**
  **67246 Dirmstein (DE)**
• **Heida, Bernd**
  **67158 Ellerstadt (DE)**

(74) Vertreter: **Ellwanger, Arndt et al**
**Ellwanger & Kern**
**Patentanwälte**
**Friedrichsplatz 9**
**68165 Mannheim (DE)**

(54) **Verfahren zur destillativen Stofftrennung eines oder mehrerer Einsatzgemische in einer Kolonne mit einer oder mehreren durchgehend angeordneten Trennwänden**

(57)    Verfahren zur destillativen Stofftrennung eines oder mehrerer Einsatzgemische in einer Kolonne mit einer, zwei oder drei in Längsrichtung der Kolonne von einem Ende bis zum anderen Ende der Kolonne durchgehend angeordneten Trennwand oder Trennwänden, die den Kolonneninnenraum in zwei, drei oder vier vollständig voneinander getrennte Kolonnenteilbereiche trennt oder trennen, mit einer gerichteten Brüdenströmungsführung aus einem ersten Kolonnenteilbereich in einen zweiten und gegebenenfalls einen dritten und gegebenenfalls einen vierten Kolonnenteilbereich sowie einer gezielten Flüssigkeitsströmungsführung im Gegenstrom zur Brüdenströmungsführung, wobei das eine oder die mehreren Einsatzgemische in der Kolonne in zwei Fraktionen aufgetrennt werden und die Kolonne einen einzigen Sumpfverdampfer und einen einzigen Kondensator am Kolonnenkopf aufweist.

FIG. 1A

**Beschreibung**

Die Erfindung betrifft ein Verfahren zur destillativen Stofftrennung eines Einsatzgemisches in einer Kolonne mit einer oder mehreren in Längsrichtung der Kolonne von einem Ende bis zum anderen Ende der Kolonne durchgehend angeordneten Trennwänden sowie eine Verwendung des Verfahrens.

Bei der Entwicklung kostengünstiger Herstellverfahren im Bereich der chemischen Industrie stellt die Prozessintegration ein wichtiges Gestaltungsprinzip dar. Dabei werden mehrere Apparate oder ganze Verfahrensstufen zu Anordnungen mit einer verringerten Anzahl von Apparaten zusammengefasst.

In der Destillationstechnik sind Trennwandkolonnen typische Beispiele für eine Prozessintegration. So ist es möglich, bei der Auftrennung von Drei-Stoff-Gemischen in reine Fraktionen die konventionellen Anordnungen von zwei separaten Destillationskolonnen oder Hauptkolonnen mit angeschlossenen Seitenkolonnen mit jeweils eigenen Verdampfern und Kondensatoren zu einer einzigen Destillationskolonne mit einer Trennwand und nur einem einzigen Verdampfer und Kondensator zusammenzufassen. Durch diese Prozessintegration können etwa 20 bis 30 % der Investitionskosten eingespart werden. Infolge der verbesserten thermodynamischen Eigenschaften mit einer Minimierung der an der Zulaufstelle auftretenden Mischungsentropie können zusätzlich Energieeinsparungen in gleicher Größenordnung erzielt werden.

Es ist auch möglich, die Auftrennung von Vier-Stoff-Gemischen zu einer einzigen Trennwandkolonne zusammenzufassen. Da bei dieser Kolonnenanordnung an der Zulaufstelle größere Mischungsentropien auftreten, die die mögliche Energieeinsparung verringern, wurde als Abhilfemöglichkeit die Anordnung einer Destillationskolonne mit mehreren Trennwänden beschrieben.

Als einfachere Möglichkeit der Prozessintegration gelten Destillationskolonnen, bei denen sich die Trennwand bis zum oberen oder unteren Ende der Kolonne erstreckt. Bei diesen Kolonnenanordnungen lassen sich ebenfalls Einsparungen an Investitionskosten erzielen. Allerdings resultieren keine Einsparungen an Energie, da diese Anordnungen lediglich eine apparative Zusammenfassung einer Hauptkolonne und einer Seitenkolonne darstellen.

Die DE-C 102 07 460 beschreibt ein Verfahren zur destillativen Auftrennung von Phthalsäureanhydrid in eine Leichtsiederfraktion, eine Schwersiederfraktion sowie eine Fraktion, enthaltend das Phthalsäureanhydrid, in einer Kolonne mit einer Trennwand, die sich über die gesamte Höhe der Kolonne erstreckt, unter Ausbildung von zwei Teilbereichen, wobei die Sümpfe der beiden Teilbereiche miteinander verbunden sind oder ein Überlauf innerhalb der Kolonne im unteren Bereich derselben ausgebildet ist, der die Trennwand unterbricht und die beiden Teilbereiche miteinander verbindet.

Trennwandkolonnen sind jedoch relativ aufwändig in der Fertigung, insbesondere muss für eine korrekte Formgebung die Trennwand spannungsfrei in den Kolonnenmantel eingeschweißt werden; hierfür ist es, insbesondere bei Kolonnen mit großem Durchmesser, erforderlich, die Trennwand in mindestens zwei, häufig sechs bis acht Schweißgängen aufwändig einzubringen. Der Einsatz von Trennwandkolonnen war daher bislang auf komplexere Trennaufgaben, insbesondere die Auftrennung von drei oder mehreren Komponenten beschränkt.

Die destillative Auftrennung von Zwei Stoff-Gemischen kann insbesondere dann aufwändig sein, wenn die relative Flüchtigkeit der Komponenten sehr gering ist. In der destillativen Trenntechnik wird in der Regel davon ausgegangen, das bei relativen Flüchtigkeiten kleiner als etwa 1,5 eine Auftrennung in der Regel nur unter Zuhilfenahme eines Lösungsmittels durchgeführt werden sollte, das die Unterschiede in den Flüchtigkeiten vergrößert. Ohne Zuhilfenahme eines Lösungsmittels wäre eine zu große Anzahl von theoretischen Trennstufen erforderlich, und entsprechend sehr große Bauhöhen der Destillationskolonne, oder anders ausgedrückt, sehr hohe Schlankheitsgrade (Verhältnis Länge : Durchmesser der Kolonne). Für den Schlankheitsgrad wird in der Regel eine Obergrenze von etwa 30 oder auch von etwa 20 angesetzt. Würden sich aufgrund der erforderlichen Trennstufenzahl höhere Schlankheitsgrade ergeben, musste die Auftrennung bislang in zwei Kolonnen durchgeführt werden.

Es war demgegenüber Aufgabe der Erfindung, ein Verfahren zur Prozessintegration bei der destillativen Auftrennung von Stoffgemischen in zwei Fraktionen zur Verfügung zu stellen, und dadurch Investitionskosten einzusparen. Insbesondere soll die Bauhöhe der Destillationskolonne verkürzt, der Schlankheitsgrad verringert und die Anzahl der benötigten Destillationskolonnen verkleinert werden.

Die Lösung besteht in einem Verfahren zur destillativen Stofftrennung eines oder mehrerer Einsatzgemische in einer Kolonne mit einer, zwei oder drei in Längsrichtung der Kolonne von einem Ende bis zum anderen Ende der Kolonne durchgehend angeordneten Trennwand oder Trennwänden, die den Kolonneninnenraum in zwei, drei oder vier vollständig voneinander getrennte Kolonnenteilbereiche trennt oder trennen, mit einer gerichteten Brüdenströmungsführung aus einem ersten Kolonnenteilbereich in eine zweiten und gegebenenfalls einen dritten und gegebenenfalls einen vierten Kolonnenteilbereich sowie einer gerichteten Flüssigkeitsströmungsführung im Gegenstrom zur Brüdenströmungsführung, wobei das eine oder die mehreren Einsatzgemische in der Kolonne in zwei Fraktionen aufgetrennt werden und die Kolonne einen einzigen Sumpfverdampfer und einen einzigen Kondensator am Kolonnenkopf aufweist.

Es wurde gefunden, dass es möglich ist, Stoffgemische mit sehr geringen relativen Flüchtigkeiten für die bislang aufgrund der sehr großen Anzahl an benötigten theoretischen Trennstufen eine Auftrennung in mindestens zwei Kolonnen oder eine Auftrennung unter Zusatz eines Lösungsmittels erforderlich war, in einer einzigen Kolonne durchzuführen, indem die Kolonne als Trennwandkolonne mit einer oder mehreren vollständig durchgezogenen Trennwänden ausgebildet wird, trotz der Vorbehalte aus der Destillationstechnik, dass es aufgrund des Temperaturprofils über die Kolonnenhöhe zu Wärmespannungen in der Trennwand kommt. Im erfindungsgemäßen Verfahren wird eine Kolonne mit einer oder mehreren in Längsrichtung der Kolonne von einem Ende bis zum anderen Ende der Kolonne durchgehend angeordneten Trennwänden eingesetzt.

Die technische Ausführung der Trennwand kann in jeder bekannten Weise erfolgen: insbesondere kann die Trennwand aus einem Blechteil bestehen, das in den Kolonnenkörper eingeschweißt oder an einem Stück eingesteckt ist, oder auch, wie in EP 1 088 577 beschrieben, aus einer Vielzahl von steck- bzw. klemmbaren Einzelteilen besteht.

Bei Stoffsystemen, bei denen sich auf beiden Seiten der Trennwand Temperaturdifferenzen von mehr als etwa 5°C einstellen, wird die Trennwand bevorzugt thermisch isolierend ausgeführt. Beispielhafte technische Ausführungen sind in EP 0 640 367 beschrieben.

Auf beiden Seiten der Trennwand können je nach Anwendungsfall unterschiedliche Drücke auftreten. Bei der Verwendung von ungeordneten Füllkörperschüttungen als Trenneinbauten sind die Trennwände durch geeignete dem Fachmann geläufige konstruktive Maßnahmen, wie beispielsweise Zuganker, zu verstärken. Bei Nutzung von geordneten Packungen oder Böden können diese zusätzlichen Maßnahmen in den meisten Anwendungsfällen unterbleiben.

Erfindungsgemäß wird das eine oder die mehreren Einsatzgemische in der Kolonne mit einer oder mehreren vollständig durchgezogenen Trennwänden in lediglich zwei Fraktionen, d.h. eine Sumpffraktion und eine Kopffraktion, aufgetrennt. Dadurch entfällt zwar der Energiegewinn durch Vermeidung von Mischungsentropie an der Zulaufstelle, wie dies der Fall bei Drei- oder Mehrstoffgemischen ist. Durch die Prozessintegration, indem die destillative Auftrennung in einer einzigen Kolonne durchgeführt wird, können jedoch etwa 20 bis 30 % der Investitionskosten eingespart werden.

Die beiden nach dem erfindungsgemäßen Verfahren abgezogenen Fraktionen können praktisch reine Fraktionen sein, das heißt jeweils einen einzigen Stoff, in einem Anteil von mindestens 90 Gew.-% oder auch von mindestens 99 Gew.-%, enthalten.

In einer Ausführungsform wird der Kolonne ein einziges Einsatzgemisch zugeführt und die Kolonne mit einer einzigen, durchgehend angeordneten Trennwand ausgestattet, die den Kolonneninnenraum in zwei vollständig voneinander getrennte Kolonnenteilbereiche trennt.

Vorteilhaft können ein oder mehrere Zwischenverdampfer und/oder ein oder mehrere Zwischenkondensatoren vorgesehen sein.

Zusätzlich zu der einen Sumpffraktion und der einen Kopffraktion können aus der Kolonne ein oder mehrere dampfförmige oder flüssige Seitenströme entnommen werden.

Bevorzugt kann eine erste Fraktion aus dem Sumpf des ersten Kolonnenteilbereichs abgezogen, der Brüdenstrom aus dem ersten Kolonnenteilbereich in den Sumpf des zweiten Kolonnenteilbereichs und der Sumpfstrom des zweiten Kolonnenteilbereichs zum Kopf des ersten Kolonnenteilbereichs geleitet und vom Kopf des zweiten Kolonnenteilbereichs eine zweite Fraktion abgezogen werden.

In einer weiteren Ausführungsform sind zwei Trennwände vorgesehen, die in Längsrichtung der Kolonne von einem Ende bis zum anderen Ende der Kolonne durchgehend angeordnet sind, und die den Kolonneninnenraum in drei vollständig voneinander getrennte Kolonnenteilbereich trennen, und wobei der Brüdenstrom aus dem ersten Kolonnenteilbereich in den Sumpf des zweiten Kolonnenteilbereichs und der Brüdenstrom aus dem zweiten Kolonnenbereich in den Sumpf des dritten Kolonnenbereichs und der Sumpfstrom aus dem dritten Kolonnenteilbereich auf den Kopf des zweiten Kolonnenbereichs sowie der Sumpfstrom aus dem zweiten Kolonnenbereich auf den Kopf des ersten Kolonnenteilbereichs geleitet werden und aus dem dritten Kolonnenteilbereich über Kopf die zweite Fraktion abgezogen wird.

Die eine oder die mehreren Trennwände können exakt in Längsrichtung der Kolonne, das heißt in der aufrechtstehenden Kolonne vertikal und parallel zueinander angeordnet sein.

Die vorliegende Formulierung, dass die Trennwand in Längsrichtung der Kolonne angeordnet ist, soll jedoch auch einschließen, dass geringfügige, insbesondere fertigungsbedingte Abweichungen von der Kolonnenlängsrichtung möglich sind.

In einer Ausführungsform kann die eine oder die mehreren Trennwände über die Kolonnenhöhe abschnittsweise versetzt angeordnet sein, so dass für jeden Kolonnenabschnitt jeweils unterschiedliche Querschnitte der durch die Trennwand voneinander getrennten Teilbereiche des Kolonneninnenraumes realisiert werden. Auf diese Weise ist es möglich, die Kolonnenteilbereiche in Längsrichtung der Kolonnen den jeweils vorliegenden Gas- und Flüssigkeitsbelastungen so anzupassen, dass, unter Berücksichtigung der gewählten Einbauten, beispielsweise Packungen oder Böden, jeweils optimale Belastungsverhältnisse gewährleistet werden.

Insbesondere können die Querschnitte der Kolonnenteilbereiche oberhalb und unterhalb von Zuläufen, Seitenabzügen, Zwischenverdampfern oder Zwischenkondensatoren jeweils unterschiedlich groß dimensioniert sein. Auch bei der Trennung von Stoffsystemen mit stark unterschiedlicher Verdampfungsenthalpie der Komponenten und der damit verbundenen starken Veränderung der Gasbelastung in Kolonnenlängsrichtung kann eine versetzte Anordnung der einen oder der mehreren Trennwände für eine optimale hydraulische Belastung vorteilhaft sein.

Der oben beschriebenen abschnittsweise versetzten Anordnung von einer oder mehreren Trennwänden entspricht bei einer konventionellen Kolonne, ohne Trennwand, ein je nach Kolonnenhöhe unterschiedlicher Kolonnendurchmesser, beispielsweise die Ausbildung einer Kolonne mit unterschiedlichem Durchmesser im Abtriebs- und Verstärkungsteil.

Bei zwei oder mehreren in Längsrichtung der Kolonne angeordneten, von einem Ende bis zum anderen Ende der Kolonne durchgezogenen Trennwände können diese in unterschiedlicher Anordnung zueinander positioniert sein: in einer Ausführungsform sind die Trennwände parallel zueinander angeordnet, in weiteren Ausführungsformen können die Trennwände konzentrisch oder schachbrettartig angeordnet sein. Die Trennwände können beispielsweise dergestalt angeordnet sein, dass sie den Kolonnenquerschnitt in Kreissegmente oder Kreissektoren aufteilen.

Gegenstand der Erfindung ist auch die Verwendung des oben beschriebenen Verfahrens zur destillativen Auftrennung eines $C_3$-Schnittes in eine Fraktion, enthaltend mindestens 70 Gew.-% Propan, insbesondere mindestens 90 Gew.-% Propan, und eine Fraktion enthaltend mindestens 99 Gew.-% Propen, insbesondere sogenannte Polymer-grade Propen, mit mindestens 99,9 Gew.-% Propen.

Gegenstand der Erfindung ist auch die Verwendung des oben beschriebenen Verfahrens zur destillativen Auftrennung eines $C_4$-Kohlenwasserstoffschnittes in eine Raffinat I-Fraktion, enthaltend Butane, Butene und maximal 2000 Gew.-ppm 1,3-Butadien, sowie einen Roh-1,3-Butadienstrom, enthaltend mindestens 98 Gew.-% 1,3-Butadien.

Die Auftrennung von $C_3$-Schnitten, das heißt von Kohlenwasserstoffströmen, enthaltend Kohlenwasserstoffe mit überwiegend drei Kohlenstoffatomen pro Moleküle, in so genannten $C_3$-Splittern, ist aufgrund der sehr geringen relativen Flüchtigkeit von Propan zu Propen sehr aufwändig. Für die rein destillative Auftrennung und einer hohen Propen-Reinheit wären eine große Anzahl, von etwa 230 Böden erforderlich, die in einer einzigen Kolonne mit technisch üblichem Schlankheitsgrad, von maximal 30, nicht untergebracht werden können. Eine derartige Kolonne wäre selbst bei geringerem Bodenabstand über 90 m hoch. Die destillative Auftrennung musste daher bislang zwingend in zwei Destillationskolonnen durchgeführt werden.

Die destillative Auftrennung unter Zusatz eines Lösungsmittels, das den Flüchtigkeitsunterschied vergrößert, durchzuführen, ist für den vorliegenden Fall ebenfalls schwierig, weil übliche polare, hochsiedende Extraktionsmittel die ungesättigten Komponenten in den Sumpf transportieren; bei der Auftrennung von Propan/Propen ist jedoch Propen, das heißt die ungesättigte Komponente, der Leichtsieder. Entsprechend würde ein übliches polares, hochsiedendes Extraktionsmittel die übliche Reihenfolge der Siedepunkte von Propan und Propen in Abwesenheit eines Extraktionsmittels umkehren, und die relative Flüchtigkeit würde in Anwesenheit eines Extraktionsmittels zunächst gegenüber einer reinen Destillation, ohne Extraktionsmittel, abnehmen.

Demgegenüber wird erfindungsgemäß ein Verfahren zur Auftrennung eines $C_3$-Schnittes zur Verfügung gestellt, das gegenüber der üblichen, einfachen Destillation vorteilhaft ist, und das ohne Zuhilfenahme eines Lösungsmittels durchgeführt werden kann.

Das erfindungsgemäße Verfahren hat gegenüber bekannten Verfahren zur destillativen Auftrennung den Vorteil, dass bei gleicher Bauhöhe wie bei Einzelkolonnen nach dem Stand der Technik eine einzige Kolonne, mit größerem Durchmesser resultiert, die bezüglich der mechanischen Stabilität, beispielsweise im Hinblick auf Windlasten und die Anfälligkeit gegen Beanspruchungen durch Erdbeben, vorteilhaft ist und somit keine teuren Verstärkungen erfordert. Darüber hinaus resultieren niedrigere Gesamtinvestitionskosten. Insbesondere werden durch Einsparen einer Kolonne auch die Kosten für das Fundament und das Stahlgerüst der Kolonne eingespart.

Bei einer Kolonne mit einer durchgezogenen Trennwand halbiert sich gegenüber einer Kolonne ohne Trennwand, bei gleicher Trennaufgabe, die Bauhöhe, bei einer Vergrößerung des der Durchmessers um den Faktor $\sqrt{2}$ .

Insbesondere fallen auch Aufwendungen für das Apparategerüst niedrig aus; dies gilt besonders, wenn die Anlage innerhalb eines geschlossenen Produktionsbaus erstellt werden muss und hohe Einzelkolonnen eine Gesamtaufstockung des Produktionsbaus erfordern würden.

Neben den wirtschaftlichen Vorteilen, die insbesondere in der Einsparung von Apparaten begründet sind, ergeben sich weitere Vorteile, insbesondere ein geringerer Platzbedarf. Durch den vergrößerten Kolonnendurchmesser der im erfindungsgemäßen Verfahren eingesetzten Trennwandkolonne reduziert sich weiterhin die Windanfälligkeit und die Erdbebenempfindlichkeit.

Die Erfindung wird im Folgenden anhand von Ausführungsbeispielen sowie einer Figur näher erläutert.

Ausführungsbeispiel 1 - Auftrennung eines $C_3$-Kohlenwasserstoffgemisches

Die Trennaufgabe besteht darin, ein $C_3$-Kohlenwasserstoffgemisch, enthaltend 93 mol-% Propen und 7 mol-% Propan in sogenanntes Polymer-grade Propen, mit 99,9 mol-% Propen aufzureinigen, wobei im Sumpfstrom ca. 10 mol-% Propen zugelassen werden.

Ausführungsbeispiel 1 - nach dem Stand der Technik

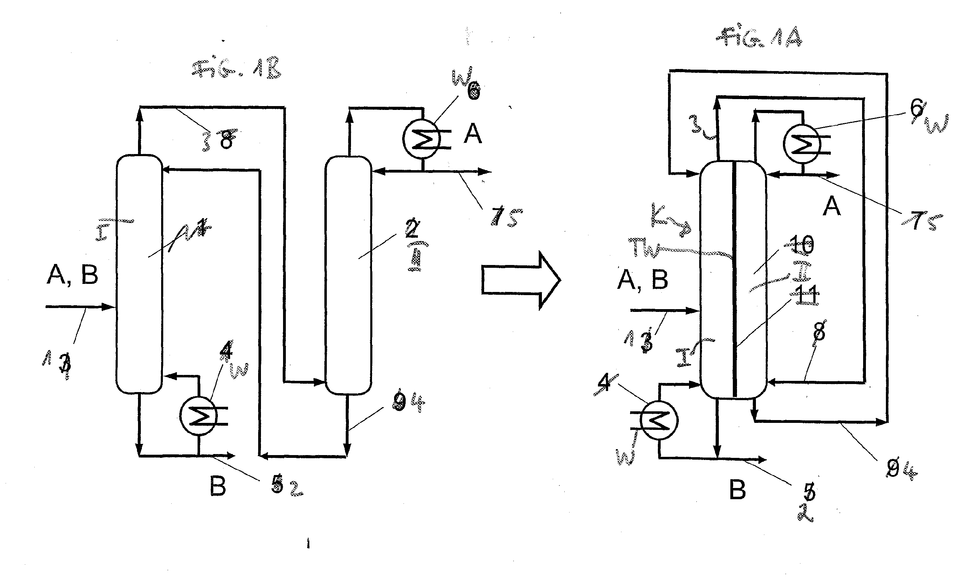

Die Auftrennung wird in einer Kolonne mit insgesamt 230 Böden, bei 18 bar Kopfdruck, die zweigeteilt, entsprechend Figur 1B gebildet ist, durchgeführt.

Der Zulauf des Feedstromes findet sich etwa in der Mitte der ersten Kolonne.

Im Sumpf der ersten Kolonne wird Propan mit ca. 10 mol-% Propen abgezogen. Der Brüdenstrom der ersten Kolonne wird in den Sumpf der zweiten Kolonne gefahren, und der Sumpfstrom der zweiten Kolonne auf den Kopf der ersten Kolonne. Am Kopf der zweiten Kolonne wird Propen mit einer Reinheit von 99,9 mol-% abgezogen. Jede der beiden Kolonnen ist ca. 50 m hoch.

Ausführungsbeispiel 1 - nach der Erfindung

Es wird eine einzige Kolonne mit durchgehender Trennwand eingesetzt. Die Kolonne ist ca. 50 m hoch, mit einem Durchmesser entsprechend etwa dem 1,4-fachen Durchmesser der Kolonne.

Ausführungsbeispiel 1 - zum Vergleich

Vom Sumpf des ersten Kolonnenteilbereichs 1 wird Propan, Strom 2, mit ca. 10 mol-% Propen abgezogen. Der Brüdenstrom, Strom 3, aus dem ersten Kolonnenteilbereich 1 wird in den Sumpf des zweiten Kolonnenteilbereichs II gefahren, und der Sumpfstrom 4 aus dem zweiten Kolonnenteilbereich II auf den Kopf des ersten Kolonnenteilbereichs I.

Ausführungsbeispiel 2 - Extraktivdestillation zur Gewinnung Rohbutadien aus einem $C_4$-Kohlenwasserstoffschnitt

Die Trennaufgabe besteht darin, Roh-1,3-Butadien aus einem $C_4$-Kohlenwasserstoffschnitt durch Extraktivdestillation zu gewinnen. Dabei wird ein $C_4$-Kohlenwasserstoffschnitt im Gegenstrom mit einem selektiven Lösungsmittel, beispielsweise Dimethylformamid oder N-Methylpyrrolidon mit 5 bis 10 Gew.-% Wasser, gewaschen, wobei der $C_4$-Kohlenwasserstoffstrom in einen Raffinatstrom, enthaltend im Wesentlichen Butane und Butene, sowie einen Roh-1,3-Butadien-Strom, mit ca. 98 Gew.-% 1,3-Butadien, aufgetrennt wird.

Ausführungsbeispiel 2 - zum Vergleich

Um die Bauhöhe der Kolonne für die Extraktivdestillation auf etwa 60 m zu begrenzen wird die Extraktivdestillation in zwei Kolonnen durchgeführt, wobei sich der Zulauf des $C_4$-Kohlenwasserstoffschnittes am Sumpf der ersten Kolonne befindet. Das selektive Lösungsmittel wird etwas unterhalb des Kopfes der ersten Kolonne zugegeben. Der Sumpfstrom der ersten Kolonne wird auf den Kopf der zweiten Kolonne gefahren, während der Brüdenstrom der zweiten Kolonne in den Sumpf der ersten Kolonne gefahren wird. Aus der zweiten Kolonne wird ein Seitenstrom gezogen und einer dritten Kolonne zugeführt, woraus durch Extraktivdestillation Gegenstrom mit einem selektiven Lösungsmittel Roh-1,3-Butadien über Kopf gewonnen wird.

Ausführungsbeispiel 2 - nach der Erfindung

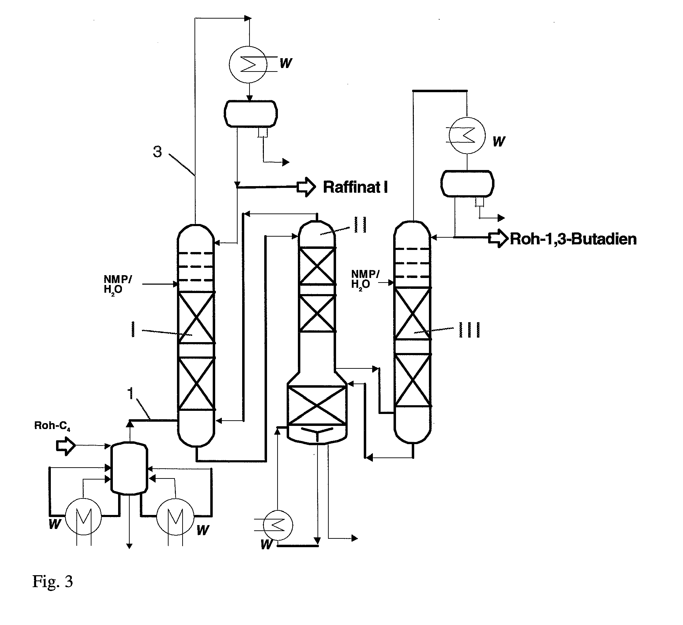

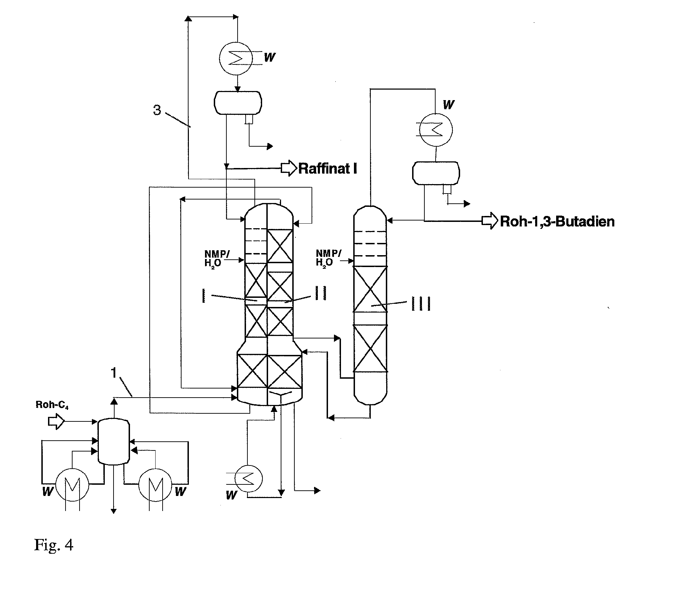

Die oben beschriebenen beiden Kolonnen werden in einem gemeinsamen Kolonnenkörper zusammengefasst und mit einer durchgehenden Trennwand voneinander getrennt, entsprechend der Darstellung in Figur 4. Die äußere Verschaltung der Kolonnenteilbereiche bleibt unverändert. Es resultiert eine einzige Kolonne, die bei gleicher Bauhöhe und größerem Durchmesser die Funktion der beiden getrennten Kolonnen 1 und II entsprechend Figur 3 erfüllt.

In einem weiteren erfindungsgemäßen Ausführungsbeispiel kann auch die dritte Kolonne III aus dem Ausführungsbeispiel 2 zum Vergleich in denselben Kolonnenkörper, unter Verwendung einer weiteren, jedoch nicht durchgehenden Trennwand, wie in DE-A 103 22 655, beschrieben integriert werden, so dass sämtliche drei, im Ausführungsbeispiel 2 zum Vergleich (Figur 3) beschriebenen Kolonnen I, II und III in einen einzigen Kolonnenkörper zusammengefasst sind.

In den Figuren bezeichnen gleiche Bezugszeichen jeweils gleiche oder entsprechende Bauteile.

Es zeigen im Einzelnen:

Figur 1A    eine erste Ausführungsform einer Trennwandkolonne zur Durchführung des erfindungsgemäßen Verfahrens,

Figur 1B    eine Darstellung der Fig. 1A entsprechenden Anordnung von zwei Destilla- tionskolonnen nach dem Stand der Technik,

Figur 2    eine weitere bevorzugte Ausführungsform einer Kolonne zur Durchführung des erfindungsgemäßen Verfahrens,

Figur 3    eine schematische Darstellung einer Anlage nach dem Stand der Technik zur Gewinnung von Roh-1,3-Butadien aus einem $C_4$- Kohlenwasserstoffschnitt, mit drei getrennten Kolonnen I bis III,

Figur 4    eine bevorzugte Ausführungsform einer Kolonne zur Durchführung des er- findungsgemäßen Verfahrens, wobei Roh-1,3-Butadien durch Extraktiv- destillation aus einem $C_4$-Schnitt gewonnen wird und

Figur 5    eine weitere bevorzugte Ausführungsform einer Kolonne zur Durchführung des erfindungsgemäßen Verfahrens, wobei Roh-1,3-Butadien durch Extrak- tivdestillation aus einem $C_4$-Kohlenwasserstoffschnitt gewonnen wird.

Figur 1A zeigt eine erste Ausführungsform einer Trennwandkolonne zur Durchführung des erfindungsgemäßen Verfahrens. Ein Einsatzgemisch, Strom 1, enthaltend die Komponenten A und B, wird auf einen ersten Kolonnenteilbereich I einer Trennwandkolonne K, mit in Längsrichtung der Kolonne K durchgehend, von einem Ende bis zum anderen Ende derselben angeordneten Trennwand TW, aufgegeben. Die Trennwand TW teilt den Innenraum der Kolonne K in einen ersten Kolonnenteilbereich I und eine zweiten Kolonnenteilbereich II. Dem ersten Kolonnenteilbereich I ist ein erster Wärmetauscher W, als Sumpfverdampfer, und dem zweiten Kolonnenteilbereich II ein zweiter Wärmetauscher W, als Kondensator am Kopf, zugeordnet.

Aus dem Sumpf des ersten Kolonnenteilbereichs I wird eine erste Fraktion, Strom 2, enthaltend die Komponente B, abgetrennt, und vom Kopf des zweiten Kolonnenteilbereichs II eine zweite Fraktion, Strom 5, enthaltend die Komponente A.

Der Brüden aus dem ersten Kolonnenteilbereich I, Strom 3, wird in den Sumpf des zweiten Kolonnenteilbereichs II, und die Sumpfflüssigkeit aus dem zweiten Kolonnenteilbereich II, Strom 4, auf den Kopf des ersten Kolonnenteilbereichs I geleitet.

Figur 1B zeigt die entsprechende Anordnung nach dem Stand der Technik, wofür zwei nebeneinander angeordnete Destillationskolonnen I und II erforderlich sind. Die Kolonne I entspricht dem ersten Kolonnenteilbereich I der erfindungsgemäß eingesetzten Kolonne K, und die Kolonne II dem zweiten Kolonnenteilbereich II der erfindungsgemäß eingesetzten Kolonne K.

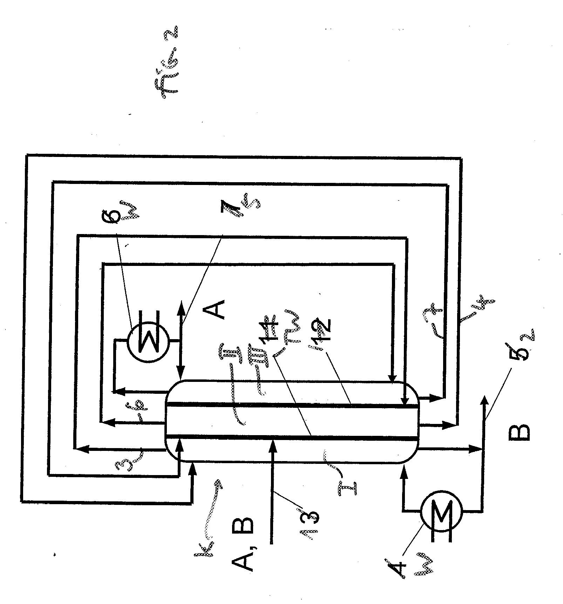

Figur 2 stellt eine weitere bevorzugte Ausführungsform für eine Kolonne zum Einsatz in einem erfindungsgemäßen Verfahren dar. Gegenüber der in Figur 1 dargestellten Ausführungsform sind in der Ausführungsform nach Figur 2 zwei Trennwände TW vorgesehen, und entsprechend wird der Innenraum der Kolonne K in drei Kolonnenteilbereich, I, II und III, aufgeteilt. Entsprechend fällt ein weiterer Kopfstrom, Strom 6, aus dem zweiten Kolonnenteilbereich II an, und wird in den Sumpf des dritten Kolonnenteilbereichs III geleitet, und ein weiterer Sumpfstrom, Strom 7, aus dem Sumpf des dritten Kolonnenteilbereichs III, der auf den Kopf des zweiten Kolonnenteilbereichs, II, aufgegeben wird.

Figur 3 zeigt eine schematische Anlage zur Gewinnung von Roh-1,3-Butadien aus einem $C_4$-Kohlenwasserstoffschnitt (Roh-$C_4$) durch Extraktivdestillation mit einem selektiven Lösungsmittel (NMP/Wasser), mit Zuführung des vorgewärmten Einsatzgemisches, Strom 1 eine erste Kolonne I, auf die im Gegenstrom das selektive Lösungsmittel NMP/Wasser aufgegeben wird, und woraus ein Brüdenstrom 3 abgezogen wird, der, nach Kondensieren in einem Wärmetauscher W als Raffinat I abgezogen wird. Der Sumpfstrom aus der ersten Kolonne I wird auf den Kopf der zweiten Kolonne II aufgegeben und der Brüdenstrom aus der zweiten Kolonne II in den Sumpf der ersten Kolonne I. Aus der zweiten Kolonne II wird ein Seitenstrom einer dritten Kolonne III zugeführt, aus der über Kopf, nach Kondensation in einem Wärmetauscher W Roh-1,3-Butadien abgezogen wird.

Figur 4 zeigt die schematische Darstellung einer Anlage zur Durchführung derselben Trennaufgabe wie in Figur 3, wobei jedoch erfindungsgemäß die beiden Kolonnen I und II in eine einzige Kolonne zusammengefasst sind, die eine über die gesamte Kolonnenhöhe vollständig durchgezogene Trennwand aufweist.

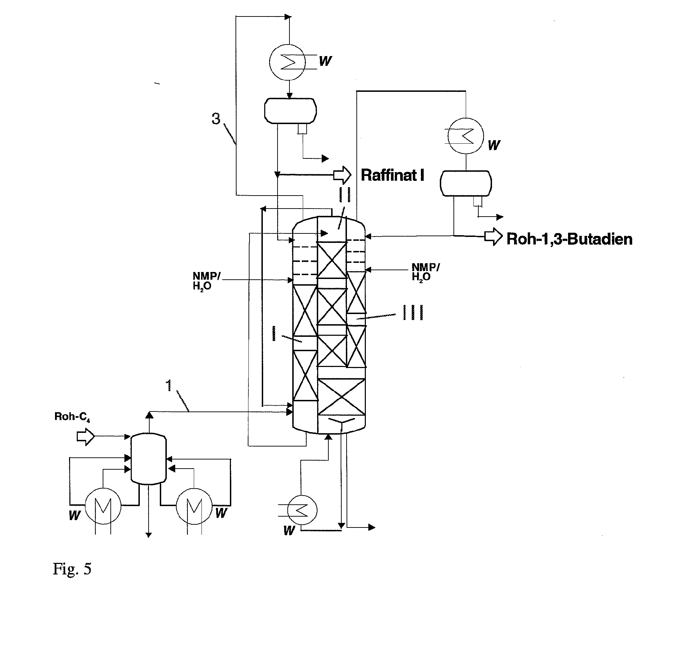

Figur 5 zeigt eine weitere bevorzugte Ausführungsform einer Kolonne nach dem erfindungsgemäßen Verfahren, wonach ebenfalls die Gewinnung von Roh-1,3-Butadien aus einem $C_4$-Kohlenwasserstoffschnitt durchgeführt wird, indem, gegenüber der in

Figur 4 dargestellten Ausführungsform zusätzlich auch die dritte Kolonne III in denselben Kolonnenkörper integriert ist.

**Patentansprüche**

1. Verfahren zur destillativen Stofftrennung eines oder mehrerer Einsatzgemische in einer Kolonne mit einer, zwei oder drei in Längsrichtung der Kolonne von einem Ende bis zum anderen Ende der Kolonne durchgehend angeordneten Trennwand oder Trennwänden, die den Kolonneninnenraum in zwei, drei oder vier vollständig voneinander getrennte Kolonnenteilbereiche trennt, mit einer gerichteten Brüdenströmungsführung aus einem ersten Kolonnenteilbereich in eine zweiten und gegebenenfalls einen dritten und gegebenenfalls einen vierten Kolonnenteilbereich sowie mit einer gerichteten Flüssigkeitsströmungsführung im Gegenstrom zur Brüdenströmungsführung, wobei das eine oder die mehreren Einsatzgemische in der Kolonne in zwei Fraktionen aufgetrennt werden und die Kolonne einen einzigen Sumpfverdampfer und einen einzigen Kondensator am Kolonnenkopf aufweist.

2. Verfahren nach Anspruch 1, **dadurch gekennzeichnet, dass** der Kolonne ein einziges Einsatzgemisch zugeführt wird und die Kolonne mit einer einzigen, durchgehend angeordneten Trennwand ausgestattet ist, die den Kolonneninnenraum in zwei vollständig voneinander getrennte Kolonnenteilbereiche trennt.

3. Verfahren nach Anspruch 1 oder 2, **dadurch gekennzeichnet, dass** ein oder mehrere Zwischenverdampfer und/oder ein oder mehrere Zwischenkondensatoren vorgesehen sind.

4. Verfahren nach einem der Ansprüche 1 bis 3, **dadurch gekennzeichnet, dass** aus der Kolonne ein oder mehrere dampfförmige oder flüssige Seitenströme entnommen werden.

5. Verfahren nach Anspruch 2, **dadurch gekennzeichnet, dass** eine erste Fraktion aus dem Sumpf des ersten Kolonnenteilbereichs abgezogen wird, der Brüdenstrom aus dem ersten Kolonnenteilbereich in den Sumpf des zweiten Kolonnenteilbereichs und der Sumpfstrom des zweiten Kolonnenteilbereichs zum Kopf des ersten Kolonnenteilbereichs geleitet und vom Kopf des zweiten Kolonnenteilbereichs eine zweite Fraktion abgezogen wird.

6. Verfahren nach einem der Ansprüche 1, 3 oder 4, **dadurch gekennzeichnet, dass** zwei Trennwände vorgesehen sind, die in Längsrichtung der Kolonne von einem Ende bis zum anderen Ende der Kolonne durchgehend angeordnet sind, und die den Kolonneninnenraum in drei vollständig voneinander getrennte Kolonnenteilbereich trennen, und aus dem dritten Kolonnenteilbereich über Kopf die zweite Fraktion abgezogen wird.

7. Verwendung des Verfahrens nach einem der Ansprüche 1 bis 6 zur destillativen Auftrennung eines $C_3$-Schnittes in eine erste Fraktion enthaltend mindestens 70 Gew.-% Propan, bevorzugt midnestens 90 Gew.-% Propan und eine zweite Fraktion enthaltend mindestens 99 Gew.-% Propen, bevorzugt mindestens 99,9 Gew.-% Propen.

8. Verwendung des Verfahrens nach einem der Ansprüche 1 bis 6 zur destillativen Auftrennung eines $C_4$-Kohlenwasserstoffschnittes in einen Raffinat I-Strom, enthaltend Butane, Butene und maximale 2000 Gew.-ppm 1,3-Butadien, sowie einen Roh-1,3-Butadienstrom, enthaltend mindestens 98 Gew.-% 1,3-Butadien.

Fig. 1B

Fig. 1A

EP 2 266 674 A1

Fig. 2

Fig. 3

Fig. 4

Fig. 5

Europäisches
Patentamt

European
Patent Office

Office européen
des brevets

# EUROPÄISCHER RECHERCHENBERICHT

**Nummer der Anmeldung**

EP 10 16 5920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2008/161618 A1 (ZIMMERMANN JOSEPH E [US] ET AL) 3. Juli 2008 (2008-07-03) * Absatz [0040] - Absatz [0044] * * Anspruch 1 * * Abbildung 3 * ----- | 1-8 | INV. B01D3/14 B01D3/32 C07C7/04 |
| A | FR 2 776 206 A1 (AIR LIQUIDE [FR]) 24. September 1999 (1999-09-24) * Ansprüche 1-10; Abbildungen 3, 4c * ----- | 5,6 | |
| A | US 4 681 661 A (GOVIND RAKESH [US]) 21. Juli 1987 (1987-07-21) * Abbildung 2 * ----- | 5 | |

| | | | RECHERCHIERTE SACHGEBIETE (IPC) |
|---|---|---|---|
| | | | B01D C07C |

Der vorliegende Recherchenbericht wurde für alle Patentansprüche erstellt

| Recherchenort | Abschlußdatum der Recherche | Prüfer |
|---|---|---|
| Den Haag | 8. November 2010 | García Alonso, Nuria |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 10 16 5920

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-11-2010

| Im Recherchenbericht angeführtes Patentdokument | Datum der Veröffentlichung | Mitglied(er) der Patentfamilie | Datum der Veröffentlichung |
|---|---|---|---|
| US 2008161618 A1 | 03-07-2008 | CN 101605873 A<br>EP 2097497 A1<br>WO 2008082981 A1 | 16-12-2009<br>09-09-2009<br>10-07-2008 |
| FR 2776206 A1 | 24-09-1999 | KEINE | |
| US 4681661 A | 21-07-1987 | CA 1220443 A1<br>EP 0140637 A1 | 14-04-1987<br>08-05-1985 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10207460 C
- EP 1088577 A
- EP 0640367 A
- DE 10322655 A